# DEMANDE DE BREVET EUROPEEN

(11) **EP 1 316 317 A1**
(43) Date de publication de la demande: **04.06.2003**
(21) Numéro de dépôt: 01204646.2
(22) Date de dépôt: 30.11.2001
(51) Int. Cl.: A61K 47/40, A61P 5/24

(54) **Associations hydrosolubles à base de cyprotérone et/ou d'un ester de celle-ci, leurs préparations et leurs utilisations**

(71) Demandeur: University of Liege, 4020 Liège (BE)
(72) Inventeur: Delattre, Luc, Université de Liège, 4000 Liège (BE); Henry de Hassonville,Sandrine, Université de Liège, 4000 Liège (BE); Evrard, Brigitte, Université de Liège, 4000 Liège (BE)

(57) **Abrégé**

Association et procédé de préparation à base de cyprotérone et/ou d'un ester de celle-ci hydrosoluble, comprenant le mélange de cyprotérone et/ou d'ester de celle-ci avec au moins une cyclodextrine choisie dans le groupe : béta-cyclodextrine et ses dérivés, la gamma-cyclodextrine et ses dérivés et leurs mélanges.

Composition pharmaceutique comprenant cette association et ayant une activité antiandrogénique et/ou progestative ou
Composition pharmaceutique ayant une activité antiséborrhéique et/ou antialopécique.

Enfin, utilisation de cette association à base de cyprotérone et/ou d'un ester en combinaison ou non avec un oestrogène, dans la préparation d'un médicament pour le traitement de l'hyperandrogénie, de l'hirsutisme, de l'acné sévère et des troubles liés à la ménopause .

Utilisation de cette association dans la préparation d'un médicament dans le traitement de la séborrhée et/ou de l'alopécie androgénique.

## Description

La présente invention concerne des associations à base de cyprotérone et/ou d'un ester de celle-ci hydrosolubles, à la préparation de telles associations et à leurs utilisations.

La cyprotérone, et plus particulièrement son ester l'acétate de cyprotérone, dérivés synthétiques de la progestérone, possèdent une activité antimaçonnique et progestative. Ils agissent par inhibition compétitive des récepteurs androgéniques. L'acétate de cyprotérone est utilisé chez l'homme dans l'hypersexualité, la puberté précoce et le cancer de la prostate. Chez la femme, il est utilisé, en association ou non avec un oestrogène, dans le traitement de l'hyperandrogénie, de l'hirsutisme, de l'acné sévère et de la séborrhée. Il est également utilisé en association dans le traitement des troubles liés à la ménopause. La solubilité de l'acétate de cyprotérone dans l'eau est très faible (environ 2 µg/ml) ce qui pourrait être en partie la cause d'une biodisponibilité très variable par voie orale et d'un manque d'action thérapeutique par voie topique. Jusqu'à ce jour, l'acétate de cyprotérone n'est utilisé que par voie orale; aucune spécialité à base de cyprotérone, d'acétate de cyprotérone avec application topique, percutanée, transcutanée ou transmuqueuse n'a été commercialisée.

La présente invention a pour but de remédier à ces inconvénients et de prévoir une association à base de cyprotérone fortement soluble dans l'eau, de stabilité accrue et dont les propriétés physico-chimiques ont été modifiées par rapport à la cyprotérone ou son ester, tout en conservant ou en diversifiant les applications pharmacologiques et/ou thérapeutiques de la cyprotérone.

A cet effet, suivant l'invention, l'association à base de cyprotérone et/ou d'un ester de celle-ci hydrosoluble comprend le mélange de cyprotérone et/ou d'ester de celle-ci avec au moins une cyclodextrine choisie dans le groupe comprenant la bêta-cyclodextrine et ses dérivés, la gamma-cyclodextrine et ses dérivés et leurs mélanges.

Suivant une autre forme de réalisation de l'invention, ladite association comprend le complexe de cyprotérone et/ou d'ester de celle-ci avec au moins une cyclodextrine choisie dans le groupe comprenant la bêta-cyclodextrine et ses dérivés, la gamma-cyclodextrine et ses dérivés et leurs mélanges.

Avantageusement, l'association comprend de l'acétate de cyprotérone.

Avantageusement, le rapport molaire cyprotérone et/ou ester de cyprotérone/cyclodextrine est de 1/1 à 1/100.

L'invention se rapporte également à la préparation du complexe à base de cyprotérone et/ou d'ester de celle-ci sous forme liquide ainsi qu'aux applications antiandrogéniques et/ou progestatives, mais aussi à des applications pour diminuer de l'alopécie et/ou de la séborrhée à partir des associations précitées.

Suivant un mode de réalisation, on agite une solution aqueuse de cyclodextrine avec un excès de cyprotérone et/ou d'ester de celle-ci jusqu'à formation d'un complexe en solution et on filtre ladite solution pour récupérer le complexe en solution dans le filtrat, la solution étant éventuellement soumise à une lyophilisation ou nébulisation pour obtenir le complexe sous forme solide.

Comme on vient de le préciser, l'association de cyprotérone et/ou de ses esters et de cyclodextrine se trouve initialement soit sous la forme d'un simple mélange soit d'un complexe formé. Lorsqu'il s'agit d'un mélange on entend par là toute association de cyprotérone/cyclodextrine donnant lieu à la formation d'un complexe lors de la dissolution du mélange dans un milieu aqueux, comme par exemple les fluides gastro-intestinaux. Pour la forme complexée, il s'agit d'une inclusion partielle ou totale de la cyprotérone ou de son ester dans la cavité de la cyclodextrine. En solution aqueuse, cette inclusion se fait partiellement, une partie de la cyprotérone restant généralement libre; il s'agit d'un équilibre. En fonction de la cyclodextrine utilisée, l'affinité de la cyprotérone pour la cyclodextrine sera différente et la partie restant libre plus ou moins importante. Cette affinité est définie par la constante d'affinité. Lorsque le complexe est sous forme solide, l'inclusion peut être totale. L'invention se rapporte à toute association de cyprotérone/cyclodextrine donnant lieu à la formation d'un complexe, le complexe pouvant déjà être formé initialement ou se former ultérieurement lors de sa dissolution en milieu aqueux. Pour une meilleure compréhension est représentée ci-après une modélisation moléculaire du complexe acétate de cyprotérone (CPA)/bêta-cyclodextrine (β-CD) : 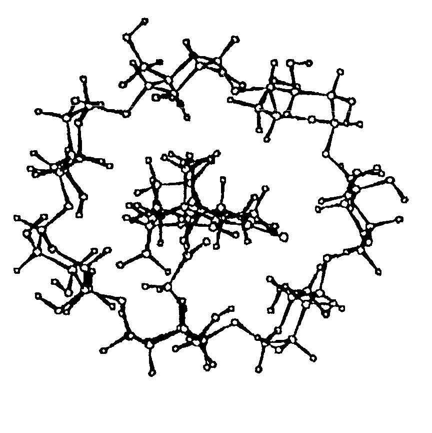 La molécule de CPA étant incluse dans la cavité de la molécule de β-CD qui se présente sous la forme d'un tore hydrophile à l'extérieur et relativement hydrophobe à l'intérieur de la cavité.

La formation de ce complexe a permis d'augmenter de façon considérable la solubilité aqueuse de la cyprotérone ou de ses esters, d'augmenter leur stabilité en fonction de la cyclodextrine utilisée et de modifier leurs propriétés physico-chimiques d'une manière générale. La biodisponibilité d'une substance étant fonction entre autres de la solubilité de cette dernière dans les fluides biologiques, l'augmentation de solubilité de la cyprotérone et de ses esters est un facteur favorisant permettant d'augmenter leur biodisponibilité, notamment par voie topique, percutanée, transcutanée, transmuqueuse ou orale. D'autre part, l'inclusion de cyprotérone dans les cyclodextrines permet d'avoir un meilleur contrôle sur sa biodisponibilité orale et de diminuer ainsi le risque d'effets secondaires.

Suivant l'invention, les cyclodextrines utilisées et permettant de former un complexe en milieu aqueux avec la cyprotérone et/ou ses esters sont la bêta-cyclodextrine et la gamma-cyclodextrine et leurs dérivés synthétiques qui sont les produits obtenus par éthérification ou estérification d'une ou plusieurs fonctions alcool libres de la cyclodextrine respective. Des exemples de dérivés de bêta-cyclodextrine sont la diméthyl bêta-cyclodextrine, l'hydroxypropyl bêta-cyclodextrine, la bêta-cyclodextrine méthylée de façon aléatoire et la sulfobutyléther 7 bêta-cyclodextrine. Des exemples de dérivés de gamma-cyclodextrine sont la diméthyl gamma-cyclodextrine, l'hydroxypropyl gamma-cyclodextrine et la gamma-cyclodextrine méthylée de façon aléatoire. On peut également utiliser des mélanges de ces bêta- et gamma-cyclodextrines et de leurs dérivés synthétiques.

Ces cyclodextrines peuvent être utilisées seules ou en association avec d'autres vecteurs tels que des liposomes, des nano- et microparticules de façon à obtenir l'effet thérapeutique désiré.

Les proportions molaires de cyprotérone et/ou ester de celle-ci/cyclodextrine varient entre 1/1 et 1/100, en fonction de la cyclodextrine utilisée et de l'utilisation que l'on veut faire du complexe.

Pour la préparation des complexes sous forme liquide on procède d'une manière générale en agitant une solution aqueuse de cyclodextrine avec un excès de cyprotérone et/ou d'ester de celle-ci jusqu'à la formation d'un complexe entre ces deux molécules. La filtration de cette solution permet de récupérer le complexe de cyprotérone-cyclodextrine en solution dans le filtrat, le complexe étant soluble dans l'eau. Le complexe peut également être obtenu en mélangeant en solution aqueuse une quantité connue de cyprotérone ou de son ester avec une quantité connue de cyclodextrine en calculant les proportions adéquates. Une autre façon d'obtenir un complexe peut consister à ajouter une solution de cyprotérone ou de son ester dans un solvant organique à une solution aqueuse de cyclodextrine. Le complexe peut se former après agitation suffisante soit après évaporation du solvant, soit même en présence du solvant. Des solvants appropriés à cet effet sont les alcools, l'acétone, les polyols tels que le propylène glycol, le glycérol et leurs mélanges.

La lyophilisation ou la nébulisation des solutions ainsi obtenues permettra d'obtenir le complexe sous forme solide. D'autres méthodes peuvent être utilisées pour la préparation de complexes solides, à savoir : agitation violente d'une suspension de la cyprotérone ou de son ester et de la cyclodextrine dans une très faible quantité d'eau puis récolte du complexe après séchage, ou bien utilisation de CO₂ à l'état supercritique : mélange de la cyprotérone ou de son ester et de la cyclodextrine en proportions adéquates en présence de CO₂ à l'état supercritique.

On donne ci-après des exemples de préparation d'associations d'acétate de cyprotérone-cyclodextrine sous forme de complexes.

Les abréviations suivantes sont utilisées :
- CPA: acétate de cyprotérone
- CD: cyclodextrine (d'une manière générale)
- βCD: bêta-cyclodextrine
- γCD: gamma-cyclodextrine
- DIMEβCD: diméthyl bêta-cyclodextrine
- DIMEγCD: diméthyl gamma-cyclodextrine
- HPβCD: hydroxypropyl bêta-cyclodextrine
- HPγCD: hydroxypropyl gamma-cyclodextrine
- RMβCD: bêta-cyclodextrine méthylée de façon aléatoire
- RMγCD: gamma-cyclodextrine méthylée de façon aléatoire
- SBEβCD: sulfobutyléther 7 bêta-cyclodextrine.

### Exemple 1

### Préparation d'un complexe CPA-CD en solution

Peser 20 mg de CPA. Ajouter 2 ml de solution d'HPβCD 200 mM. Agiter pendant 24 heures à 25°C. Filtrer sur filtre millipore Millex HV 0,45 µm. La solution obtenue après filtration contient le complexe CPA-CD en solution.

### Exemple 2

### Préparation d'un complexe CPA-CD en solution

Peser 5 mg de CPA. Ajouter 2 ml de solution d'HPβCD 200 mM. Agiter à 25°C pendant 24 heures ou jusqu'à dissolution complète du CPA. La solution ainsi obtenue contient le complexe CPA-CD.

En lyophilisant le complexe sous forme liquide et au départ des mêmes quantités de CPA (5 mg) et d'HPβCD (200 mM) (rapport molaire 1/33) on obtient le complexe sous forme solide.

### Exemple 3

### Preuves d'inclusion de CPA dans les complexes sous forme liquide : diagramme de solubilité

Lors de la formation d'un complexe, le CPA pratiquement insoluble dans l'eau (<3 µg/ml) se solubilise de façon plus ou moins importante en fonction de la concentration et de la nature des CD. L'augmentation de solubilité du CPA est donc une preuve de la formation d'un complexe entre le CPA et la CD.

Les diagrammes de solubilité sont effectués en ajoutant un excès de CPA à des solutions de CD de concentration croissante. Après 24 heures d'agitation dans des bains thermostatisés à 25°C, ces solutions sont filtrées et la quantité de CPA solubilisée est dosée par HPLC.

La βCD et la γCD ainsi que leurs dérivés synthétiques ont permis de former des complexes avec l'acétate de cyprotérone.

La β-CD provient de chez Colloïdes Naturels International (CNI), l'HPβCD et la SBEβCD proviennent d'échantillons de chez Janssens, et la RMβCD, la γCD et la HPγCD ont été fournies par Wacker.

### Préparation de solutions aqueuses de CD :

- βCD : solutions contenant 5,10,15 mM.
- HPβCD : solutions contenant 12,5; 25; 50; 100; 200 mM.
- SBEβCD : solutions contenant 12,5; 25; 50; 100; 200 mM.
- RMβCD : solutions contenant 12,5; 25; 50; 100; 200 mM.
- γCD : solutions contenant 12,5; 25; 50; 100; 150 mM.
- HPγCD : solutions contenant 12,5; 25; 50; 100; 200 mM.

### Formation des complexes :

Pesée de minimum 30 mg de CPA et ajout de 2 ml de solution de CD. Pour chaque concentration de CD, les essais sont répétés au moins deux fois. Des essais sont également effectués avec une solution aqueuse sans CD.

Les échantillons sont mis en agitation dans des bains thermostatisés à 25°C pendant 24 heures. Ensuite, ces échantillons sont filtrés sur filtre en polyvinyldifluoridène (PVDF) de 0,45 µm (Millipore, Millex HV 0,45 µm) et la concentration de CPA dans les filtrats est dosée par HPLC.

### Dosage du CPA : Méthode HPLC

### Appareillage :

- Echantillonneur automatique Merck L-7200, Détecteur UV Merck L-7400, Pompe Merck L-7100 et intégrateur Merck D-2500.

### Conditions chromatographiques :

- Colonne : Lichrospher RP18 (5 µm) 125 mm
- Phase mobile : ACN/H₂O 50/50
- Débit : 1,5 ml/min
- Température : 25°C
- Longueur d'onde : 282 nm
- Etalon interne : propylparaben.

### Résultats :

Les résultats des dosages HPLC du CPA sont repris dans les Tableaux ci-après (Tableaux 1 à 6) pour chaque cyclodextrine. Sans cyclodextrine, la solubilité obtenue est de 2,6 µg/ml.

**Tableau 1 :**

| **Solubilité du CPA en présence de βCD** | |
|---|---|
| Concentration en CD en mM | Concentration en CPA en µg/ml |
| 5 | 28,8 |
| 10 | 56,7 |
| 15 | 86,5 |

**Tableau 2 :**

| **Solubilité du CPA en présence de HPβCD** | |
|---|---|
| Concentration en CD en mM | Concentration en CPA en µg/ml |
| 12,5 | 147,1 |
| 25 | 308,0 |
| 50 | 629,9 |
| 100 | 1188,6 |
| 200 | 2648,2 |

**Tableau 3 :**

| **Solubilité du CPA en présence de SBEβCD** | |
|---|---|
| Concentration en CD en mM | Concentration en CPA en µg/ml |
| 12,5 | 243,9 |
| 25 | 461,8 |
| 50 | 860,9 |
| 100 | 1475,2 |
| 200 | 2034,1 |

**Tableau 4 :**

| **Solubilité du CPA en présence de RMβCD** | |
|---|---|
| Concentration en CD en mM | Concentration en CPA en µg/ml |
| 12,5 | 176,9 |
| 25 | 430,9 |
| 50 | 1169,9 |
| 100 | 3222,1 |
| 200 | 9598,7 |

| **Tableau 5 : Solubilité du CPA en présence de γCD** | |
|---|---|
| Concentration en CD en mM | Concentration en CPA en µg/ml |
| 12,5 | 195,2 |
| 25 | 436,6 |
| 50 | 1041,0 |
| 100 | 2368,9 |
| 200 | 2266,8 |

**Tableau 6 :**

| **Solubilité du CPA en présence de HPγCD** | |
|---|---|
| Concentration en CD en mM | Concentration en CPA en µg/ml |
| 12,5 | 118,7 |
| 25 | 282,5 |
| 50 | 578,9 |
| 100 | 1512,0 |
| 200 | 4291,1 |

L'acétate de cyprotérone forme des complexes avec toutes les cyclodextrines étudiées car on observe dans tous les cas une augmentation de la solubilité.

La βCD naturelle présente les résultats les moins intéressants en raison de sa faible sensibilité mais par contre, ses dérivés synthétiques permettent d'augmenter la solubilité de l'acétate de cyprotérone de façon importante. La γCD naturelle ainsi que ses dérivés montrent également des résultats intéressants.

Le Tableau 7 résume les résultats de solubilité obtenus pour chaque cyclodextrine à la concentration maximale testée. L'augmentation de solubilité est calculée par rapport à la solubilité de l'acétate de cyprotérone dans l'eau (en l'absence de cyclodextrine) qui a été déterminée à 2,6 µg/ml.

Les augmentations de solubilité les plus importantes sont obtenues avec la RMβCD, la HPβCD et la HPγCD.

**Tableau 7**

| **Augmentation maximale de solubilité du CPA obtenue pour chaque cyclodextrine.** | | | |
|---|---|---|---|
| | Concentration maximale utilisée en mM pour chaque CD | Solubilité maximale en µg/ ml | Augmentation de solubilité |
| βCD | 15 | 86,5 | 33 x |
| HPβCD | 200 | 2648,2 | 1018 x |
| SBEβCD | 200 | 2034,1 | 782 x |
| RMβCD | 200 | 9598,7 | 3692 x |
| γCD | 150 | 2266,8 | 872 x |
| HPγCD | 200 | 4291,1 | 1650 x |

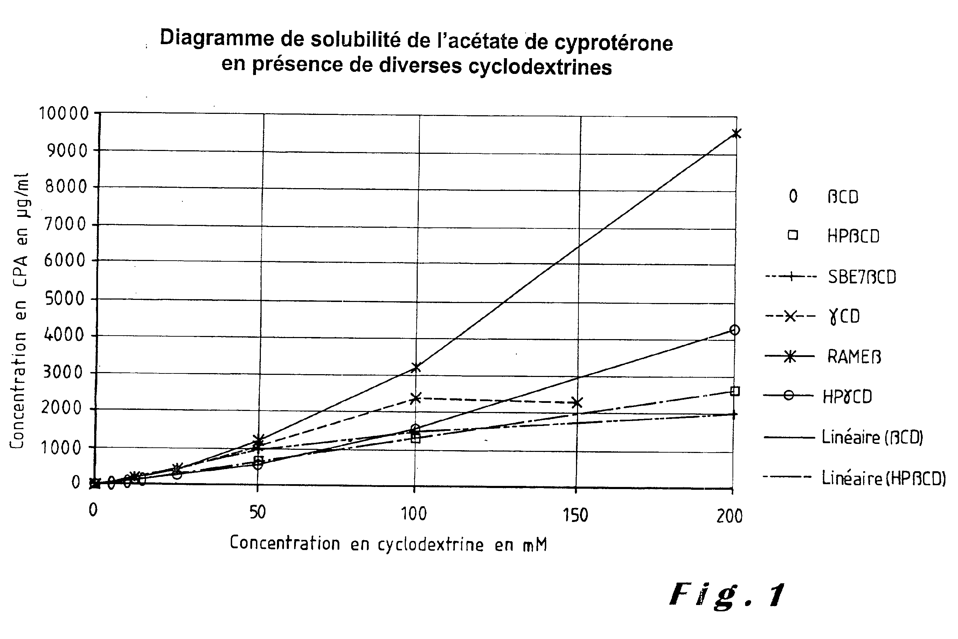

La figure 1 résume les résultats de solubilité sous forme de graphique. Ce diagramme montre que l'allure des courbes est différente en fonction des cyclodextrines.

En effet, pour la βCD et l'HPβCD, la concentration en CPA évolue de façon linéaire avec la concentration en cyclodextrine. Selon la classification de Higuchi et Connors (Adv. Anal. Chem. And Instr., 1965, 4, 117-212), ces diagrammes correspondent donc à des diagrammes de type A_{L}. Il y a formation d'un complexe soluble dans l'eau de stoechiométrie 1/1. Les constantes de stabilité de ces complexes ont donc pu être calculées et sont de 4675 M⁻¹ pour la βCD et de 10989 M⁻¹ pour l'HPβCD.

Par contre, pour les autres cyclodextrines, la relation n'est pas linéaire et les constantes de stabilité n'ont pas pu être calculées.

Pour la γCD, une précipitation du complexe est observée à partir d'une concentration de 100 mM. Pour la SBEβCD, le diagramme obtenu est de type A_{N}, tandis que pour la RMβCD et l'HPγCD, les diagrammes sont de type A_{P}.

Comme on peut le remarquer, les digrammes obtenus sont de types très différents en fonction des cyclodextrines; il n'est donc pas possible de prédire ce qui se passera pour d'autres cyclodextrines. Par contre, on peut conclure que la cavité de βCD et de la γCD ont toutes les deux une taille adéquate pour l'inclusion de l'acétate de cyprotérone.

### Exemple 4

### Etudes RMN

Des études RMN protoniques ont permis de montrer également l'inclusion du CPA dans les CD.

### Solution de référence en DIMEβCD :

Solution de DIMEβCD à 10 mM dans D₂O.

### Préparation d'un complexe sous forme liquide :

Ajout de 20 mg de CPA à 2 ml de solution de DIMEβCD à 10 mM dans D₂O. Agitation pendant 24 heures à 25°C. Filtration sur filtre Millex HV 0,45 µm.

La comparaison des graphiques de la solution de référence et du complexe CPA-DIMEβCD montre un déplacement des H en 3 et en 5 de la DIMEβCD, ce qui montre qu'il y a une interaction entre la CD et le CPA.

### Exemple 5

### Augmentation de stabilité du CPA en solution aqueuse

La formation de complexe entre le CPA et les CD peut modifier les propriétés physico-chimiques du CPA et notamment augmenter la stabilité de ce dernier.

### 1) Complexe avec la βCD

La βCD utilisée provient de chez CNI.

Un complexe est formé par ajout de 2 ml de solution aqueuse de βCD 10 mM à 20 mg de CPA. Après agitation pendant 24 heures à 25°C, ces solutions sont filtrées sur filtre en PVDF de 0,45 µm (Millipore, Millex HV 0,45 µm). La formation du complexe est effectuée sur 8 échantillons différents.

Les solutions de référence de CPA sont préparées en ajoutant 2 ml de solution aqueuse à 20 mg de CPA. Après agitation pendant 24 heures à 25°C, ces solutions sont filtrées sur filtre en PVDF de 0,45 µm. 8 échantillons sont préparés.

Les échantillons sont conservés dans des conditions ambiantes (20°C) pendant 3 mois.

La concentration en CPA contenue dans les solutions de CPA seul ou sous forme de complexe est dosée par HPLC au temps 0, après 1 mois et après 3 mois.

L'appareillage et les conditions chromatographiques sont ceux tels qu'utilisés dans l'Exemple 3.

Comme le montrent les résultats du Tableau 8, le CPA s'est révélé plus stable lorsqu'il était sous forme de complexe avec la βCD.

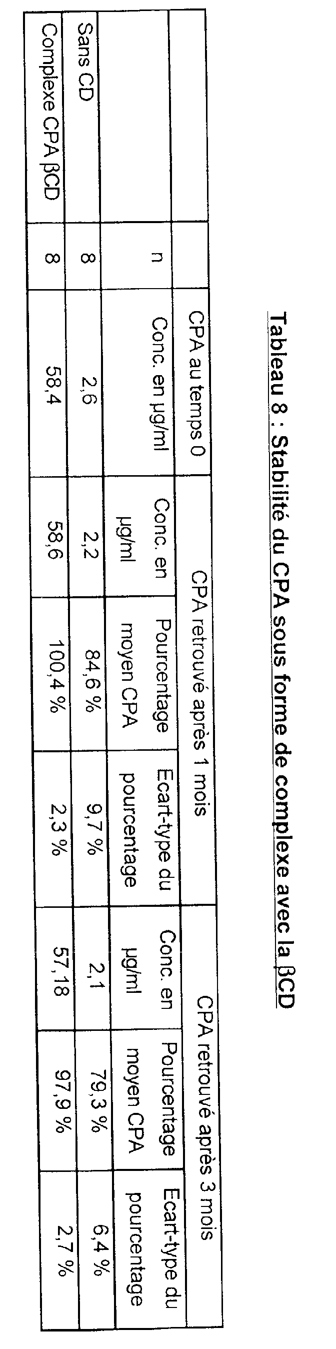

En effet, la solution de CPA sans CD semble se dégrader déjà après 1 mois ce qui a pu être confirmé par l'analyse statistique, les valeurs de concentration en CPA étant significativement différentes au temps 0 et au temps 1 mois (p<0,05).

Par contre, pour le complexe, il n'y a pas de différence significative entre les valeurs de concentrations même après 3 mois (P=0,1).

En comparant le complexe à la solution de CPA, déjà après 1 mois, l'analyse statistique de ces résultats montre que le pourcentage de CPA (84,6 %) retrouvé dans la solution de CPA seul est significativement différent du pourcentage de CPA (100,4%) retrouvé dans le complexe (p<0,05).

### 2) Complexe avec la RMβCD

La RMβCD utilisée provient de Wacker.

Les complexes sont formés par ajout de 2 ml de solution aqueuse de RMβCD à 30 mg de CPA. Des solutions de RMβCD à différentes concentrations ont été utilisées: 12,5; 25; 50; 100 et 200 mM. Après agitation pendant 24 heures à 25°C, ces solutions sont filtrées sur filtre en PVDF de 0,45 µm (Millipore, Millex HV 0,45 µm).

Les solutions de référence de CPA sont préparées en ajoutant 2 ml de solution aqueuse à 30 mg de CPA. Après agitation pendant 24 heures à 25°C, ces solutions sont filtrées sur filtre en PVDF de 0,45 µm (Millipore, Millex HV 0,45 µm).

Les échantillons sont conservés dans des conditions ambiantes (20°C) pendant 3 mois.

La concentration en CPA contenu dans les solutions de CPA seul ou sous forme de complexe est dosée par HPLC au temps 0, après 1 mois et après 3 mois.

Les conditions chromatographiques sont telles qu'utilisées précédemment.

Les complexes formés avec la RMβCD semblent légèrement moins stables que ceux formés avec la βCD et la concentration en RMβCD semble avoir peu d'influence sur la stabilité (voir Tableau 9).

Néanmoins, comme pour la βCD, la formation du complexe CPA- RMβCD augmente de façon significative la stabilité de la solution aqueuse de CPA seul (p<0,05).

**Tableau 9**

| **Stabilité du CPA sous forme de complexe avec la RMβCD** | | | | | | |
|---|---|---|---|---|---|---|
| | n | Conc. t0 en µg/ml | Conc. t1 en µg/ml | Pourcentage t1 mois | Conc. t0 en µg/ml | Pourcentage t3 mois |
| Sans CD | 4 | 2,7 | 2,2 | 83,7% | 2,1 | 77,1 % |
| RMβCD 12,5mM | 1 | 181,0 | 173,8 | 96,0% | 171,3 | 94,6% |
| RMβCD 25 mM | 1 | 430,9 | 417,8 | 97,0% | 418,3 | 97,0% |
| RMβCD 50mM | 2 | 1169,9 | 1121,4 | 95,9% | 1078,5 | 92,2 % |
| RMβCD 100 mM | 2 | 3222,1 | 3071,6 | 95,4% | 3008,2 | 93,4% |
| RMβCD 200 mM | 2 | 9598,7 | 9169,2 | 95,5% | 8995,9 | 93,7% |

### Exemple 6

### Augmentation de stabilité du CPA en milieu alcalin (conditions de stress)

L'inclusion de l'acétate de cyprotérone dans des cyclodextrines peut augmenter sa stabilité en solution aqueuse, également en condition de stress.

L'HPβCD provient d'échantillons de chez Janssen, la RMβCD et l'HPγCD ont été fournies par Wacker.

Les complexes sont préparés en ajoutant 2 ml de solution aqueuse de CD à 20 mg de CPA. Les solutions de CD utilisées pour préparer ces complexes sont des solutions de HPβCD, de HPγCD ou de RMβCD à une concentration de 100 mM. Après agitation pendant 24 heures à 25°C, ces solutions sont filtrées sur filtre en PVDF de 0,45 µm (Millipore, Millex HV 0,45 µm).

Les solutions de référence de CPA sont préparées en ajoutant 2 ml de solution aqueuse à 20 mg de CPA. Après agitation pendant 24 heures à 25°C, ces solutions sont filtrées sur filtre en PVDF de 0,45 µm (Millipore, Millex HV 0,45 µm).

Chaque essai est répété à 3 reprises et les échantillons sont conservés dans des étuves à température contrôlée à 25°C pendant 1 mois.

La concentration en CPA contenu dans les solutions de CPA seul ou sous forme de complexe est dosée par HPLC au temps 0 et après 1 mois et le pH des solutions est mesuré après 1 mois.

Les conditions chromatographiques sont telles qu'utilisées précédemment.

Après 1 mois, la libération de substances alcalines provenant des flacons a communiqué un pH alcalin aux solutions. Ce pH a été mesuré et varie entre 8 et 9 dans les différentes solutions. Ce pH alcalin a provoqué la dégradation de l'acétate de cyprotérone, par hydrolyse de la fonction ester.

La concentration en CPA a donc été mesurée par HPLC et les résultats sont donnés dans le Tableau 10.

**Tableau 10**

| **Stabilité en milieu alcalin du CPA seul ou complexé avec diverses CD** | | | | |
|---|---|---|---|---|
| | Conc. t0 en µg/ml | Conc. t1 mois en µg/ml | Pourcentage CPA après 1 mois | Ecart-type |
| Sans CD | 2,3 | 0,4 | 17,6 % | 3,7% |
| HPβCD | 1243,0 | 836,2 | 67,3 % | 0,6% |
| HPγCD | 1524,7 | 913,9 | 59,9 % | 6,7% |
| RMβCD | 2915,3 | 2541,3 | 87,2 % | 1,9% |

Ces résultats montrent que les trois cyclodextrines étudiées, l'HPβCD, l'HPγCD et la RMβCD augmentent de façon spectaculaire la stabilité du CPA en milieu alcalin. En effet, alors que la quantité de CPA retrouvé après 1 mois n'est plus que de 17 % dans les échantillons sans cyclodextrine, elle est supérieure à 50 % pour les complexes. Les résultats les plus impressionnants sont obtenus avec la RMβCD pour laquelle on récupère près de 90 % du CPA.

### Exemple 7

### Autre propriété des complexes CPA-CD : Diminution de l'absorption du CPA sur la cellulose

Lors des recherches effectuées, on a pu remarquer que l'acétate de cyprotérone avait tendance à s'adsorber sur les filtres de cellulose. D'autre part, la présence de cellulose microcristalline dans la formulation de comprimés contenant de l'acétate de cyprotérone semble freiner la libération de cette substance active dans le milieu de dissolution.

L'acétate de cyprotérone est donc une molécule qui s'adsorbe facilement sur la cellulose. La formation de complexe avec les cyclodextrines permet de diminuer considérablement cette adsorption sur la cellulose ce qui peut être intéressant pour des formulations contenant de la cellulose ou éventuellement d'autres produits susceptibles d'adsorber le CPA.

Cette diminution d'adsorption du CPA sur la cellulose, en association avec l'augmentation de solubilité et de stabilité, montre que la formation de complexes avec les cyclodextrines change les propriétés physico-chimiques de l'acétate de cyprotérone.

La β-CD provient de chez CNI, l'HPβCD et la SBEβCD proviennent d'échantillons de chez Janssen, et la RMβCD, la γCD et la HPγCD ont été fournies par Wacker.

Les complexes ont été préparés en diluant 50 fois une solution mère de CPA à 100 µg/ml dans un mélange eau/acétonitrile (ACN) 50/50, avec une solution aqueuse de cyclodextrine à 10 mM. Les différentes cyclodextrines utilisées sont la β-CD, la γCD, l'HPβCD, l'HPγCD et la RMβCD. Ces échantillons sont agités au vortex pendant au moins 1 minute. Etant donné la faible proportion de CPA par rapport à la concentration en cyclodextrine et le CPA étant déjà solubilisé dans le milieu, il n'est pas nécessaire d'agiter 24 heures à 25°C pour obtenir le complexe, ce dernier étant formé très rapidement.

Une solution de référence contenant seulement du CPA est préparée en diluant 50 fois la solution mère de CPA à 100 µg/ml dans un mélange eau/acétonitrile 50/50, avec de l'eau purifiée.

Chaque essai est répété à trois reprises. Ces échantillons sont ensuite filtrés en partie sur filtre en ester de cellulose et en partie sur filtre en PVDF. Les échantillons sont dosés avant et après filtration de façon à pouvoir calculer le pourcentage de récupération après filtration.

Les résultats repris dans le Tableau 11 montrent une adsorption importante du CPA après la filtration d'une solution aqueuse sans cyclodextrine sur un filtre en ester de cellulose. En effet, la quantité de CPA récupéré après filtration par rapport à la quantité présente avant filtration est très faible (moins de 5 %). Par contre, lors de la filtration sur filtre en PVDF, le CPA n'est pratiquement pas adsorbé, on récupère près de 90 %.

Lorsque l'on ajoute des cyclodextrines, en fonction des cyclodextrines ajoutées, la quantité de CPA récupéré après filtration sur la cellulose va de 45 à 75 %, ce qui est nettement plus important que la quantité récupérée après filtration sans cyclodextrine, et la totalité du CPA est récupérée après filtration sur PVDF.

**Tableau 11**

| **Pourcentage de CPA récupéré après filtration sur filtre en cellulose et en PVDF.** | | | | |
|---|---|---|---|---|
| | Pourcentage de CPA récupéré après filtration sur acétate de cellulose | | Pourcentage de CPA récupéré après filtration sur PVDF | |
| | Moyenne | Ecart-type | Moyenne | Ecart-type |
| Sans CD | 2,9 % | 5,1 % | 88,7 % | 6,9 % |
| βCD | 50,1 % | 3,0 % | 99,2 % | 3,4 % |
| γCD | 66,5 % | 9,7 % | 101,4 % | 7,1 % |
| HPβCD | 65,9% | 11,3% | 100,3% | 1,0 % |
| HPγCD | 47,5 % | 2,6 % | 93,2 % | 4,3 % |
| RMβCD | 64,8 % | 2,0 % | 98,0 % | 0,8 % |

La formation d'un complexe avec les cyclodextrines permet donc de diminuer de façon conséquente l'adsorption du CPA sur la cellulose.

Les associations cyprotérone-cyclodextrine de l'invention peuvent être administrées en combinaison avec divers autres excipients pharmaceutiques, tels que diluants, gélifiants, agents conservateurs, émulsionnants, édulcorants, aromatisants et cela par voie orale, topique ou autre.

Pour une administration orale, on utilisera des comprimés, gélules, poudre, sirops, solutions, ou d'autres formulations galéniques. Ces formes galéniques peuvent libérer le principe de façon normale ou programmée dans le temps. Toutefois, les applications thérapeutiques de l'acétate de cyprotérone nécessitant généralement la présence d'un taux sanguin constant en CPA, les formes galéniques qui seront les plus adéquates pour la voie orale sont donc les comprimés et les gélules.

Le composé actif peut être administré seul ou en combinaison avec d'autres produits actifs ayant une activité similaire ou différente.

On donne ci-après quelques exemples non limitatifs de préparation de formulations de l'invention comprenant du CPA et une cyclodextrine sous forme de complexe ou en mélange.

### 1) Formulation à usage oral

### Comprimés contenant 2 mg de CPA et 0,035 mg d'éthinyloestradiol

### Composition

Pour des comprimés de 350 mg :
- Complexe CPA-HPβCD217 mg (équivalent à 2 mg de CPA)
- Ethinyloestradiol 0,035 mg
- Lactose monohydraté 122,2 mg (diluant)
- Carboxyméthylamidon 7 mg (désintégrant, Explotab®)
- Stéarate de magnésium 2 mg (lubrifiant)
- Silice colloïdale 1,75 mg (régulateur d'écoulement, Aerosil®

### Mode opératoire

Pour 3,5 kg de comprimés (environ 10.000 comprimés) :
a) Préparation du complexe : Peser 2688 g de HPβCD et les dissoudre dans 10 litres d'eau. Ajouter 25 g de CPA et agiter jusqu'à dissolution complète de ce dernier (laisser agiter pendant 24 heures à 25°C). Lyophiliser la solution ainsi obtenue.
b) Préparation des comprimés : Peser 350 mg d'éthinyloestradiol et les mélanger avec 25 g de lactose monohydraté et 1,5 g de silice colloïdale dans un mélangeur TURBULA. Tamiser ce mélange sur un tamis de 0,5 mm. Ajouter le reste du lactose (1197 g), le complexe HPβCD-CPA (2170 g), le carboxyméthylamidon (70 g) et le reste de silice colloïdale (16 g). Mélanger pendant 5 minutes dans un mélangeur LÖDIGE. Ajouter 20 g de stéarate de magnésium et mélanger pendant 30 secondes. Fabriquer des comprimés pesant théoriquement 350 mg.

### 2) Formulations à usage topique, percutané, transcutané ou transmuqueuse

Différentes formes galéniques peuvent être envisagées pour les voies topique, percutanée, transcutanée ou transmuqueuse : crèmes hydrophiles ou lipophiles, gels, lotions, suspensions, etc.

Pour une application dans l'acné par exemple, la formulation d'un gel pourrait être une forme agréable à appliquer sur la peau.

### a) Préparation d'un gel aqueux à 1 % de CPA.

### Composition

Pour 100 g de gel :
- CPA 1 g
- RMβCD 26,9 g (200 mM)
- Sorbate de potassium 0,27 g (conservateur)
- Phosphate monosodique dihydraté 0,3 g (tampon)
- Tylose® H4000 3 g (hydroxyéthylcellulose, gélifiant)
- Eau purifiée q.s. pour faire 100 g.

### Mode opératoire

a) Peser 26,9 g de RMβCD (2,46 % d'H₂O, Wacker) et les dissoudre dans 60 ml d'eau purifiée fraîchement bouillie et refroidie.
b) Peser 1 g d'acétate de cyprotérone et 0,3 g de phosphate monosodique dihydraté et les ajouter à la solution (a) en agitant vigoureusement de façon à dissoudre un maximum de CPA.
c) Disperser petit à petit les 3g d'hydroxyéthylcellulose (Tylose® H4000) dans la suspension (b).
d) Dissoudre 0,27g de sorbate de potassium dans quelques millilitres d'eau purifiée fraîchement bouillie et refroidie et ajouter cette solution aqueuse au gel (c).
e) Compléter avec de l'eau de façon à obtenir 100 g de gel.
f) Laisser reposer 24 heures pour permettre la solvatation du polymère.

Il est normal que toute la quantité de CPA ne soit pas dissoute étant donné que la quantité de RMβCD est insuffisante. La présence d'un léger excès de CPA est intéressante pour former un gradient de concentration qui va augmenter la pénétration du CPA dans la peau.

### b) Crème hydrophile à 0,2 %

### Composition

Pour 100 g de crème :
- Complexe CPA-HPγCD 17,315 g (équivalent à 0,2 g CPA)
- Crème au cétomacrogol tamponnée 60 g
- Eau purifiée q.s. pour faire 100 g

Composition de la crème au cétomacrogol :
- Alcool cétostéarylique 6 g
- Cétomacrogol 1000 1,5 g
- Vaseline blanche 12 g
- Paraffine liquide 5 g
- Sorbate de potassium 0,22 g
- Phosphate monosodique dihydraté 0,25 g
- Eau purifiée q.s. pour faire 60 g.

### Mode opératoire

a) Préparation du complexe : Peser 34,23 g d'HPβCD (7,92 % H₂O, Wacker) et 0,4 g de CPA, ajouter de l'eau petit à petit en agitant suffisamment de façon à dissoudre entièrement les deux substances et jusqu'à atteindre 100 ml de solution. Cette solution atteint une concentration en HPγCD égale à 200 mM. Lyophiliser la solution de façon à obtenir le complexe sous forme solide.
b) Préparation de la crème : Faire fondre dans une capsule à 70°C 6 g d'alcool cétostéarylique, 1,5 g de cétomacrogol 1000,12 de vaseline blanche et 5 g de paraffine liquide. Faire bouillir 40 g d'eau purifiée, en prélever 25g et y dissoudre 0,25g de phosphate monosodique dihydraté. Quand la température de la phase aqueuse atteint 70°C, l'incorporer sous agitation à la phase grasse fondue et réaliser l'émulsion à l'aide d'un disperseur-homogénéisateur. Laisser refroidir en agitant constamment. Dissoudre 0,22 g de sorbate de potassium dans 5 g d'eau purifiée fraîchement bouillie et refroidie et ajouter cette solution à la crème refroidie. Amener si nécessaire à pH 5 par addition soit d'acide phosphorique dilué soit d'une solution d'hydroxyde de sodium diluée. Ajuster à 60 g avec de l'eau purifiée fraîchement bouillie et refroidie.
c) Peser 17,315g du complexe (a) (0,2g de CPA + 17,115 g d'HPγCD). Porphyriser dans un mortier avec 20 ml d'eau purifiée. Une partie du complexe va se dissoudre. Ajouter petit à petit la crème (b) en y incorporant le complexe. Ajuster à 100 g avec de l'eau purifiée.

### Solution à usage transnasal à 0,9 % CPA

### Composition

Pour 30 ml de solution pour pulvérisation nasale :
- CPA 270 mg
- RMβCD 9 g (200 mM)
- Chlorure de sodium 270 mg
- HCI ad pH 6
- Eau purifiée ad 30 ml

### Mode opératoire:

a) Peser 0,27 g d'acétate de cyprotérone, 0,27g de chlorure de sodium et 9 g de RMβCD (2,46% d'H₂O, Wacker).
b) Dissoudre les différents constituants dans 20 ml d'eau purifiée préalablement bouillie et refroidie.
c) Ajuster au pH de 6 à l'aide HCI dilué (1N).
d) Ajuster au volume de 30 ml avec de l'eau purifiée préalablement bouillie et refroidie

Différents autres types de formulations peuvent être envisagées :
- Formulations à usage transdermique
- Formulations à usage parentéral
- Dispositif intra-utérin.

Comme on l'a déjà précisé, des associations avec d'autres systèmes peuvent également être envisagées, comme les nano- ou microparticules ou les liposomes par exemple.

Les associations antiandrogéniques et progestatives de l'invention ont les mêmes applications que l'acétate de cyprotérone utilisé seul et sont par conséquent plus particulièrement actives chez l'homme dans l'hypersexualité, la puberté précoce et le cancer de la prostate et chez la femme, en association ou non avec un oestrogène, dans le traitement de l'hyperandrogénie, de l'hirsutisme, de l'acné sévère et des troubles liés à la ménopause. En outre, ces associations peuvent également être utilisées dans de nouvelles applications, telles que, pour le traitement de la séborrhée chez la femme et éventuellement chez l'homme, par exemple par voie topique si le passage de l'acétate de cyprotérone dans la circulation systémique est limitée. Enfin ces associations peuvent être utilisées dans de nouvelles applications pour le traitement de l'alopécie ou le traitement de toute autre affection liée à une quantité trop élevée d'androgènes ou à un nombre important de récepteurs androgéniques ou à une sensibilité accrue de ces récepteurs androgéniques.

## Revendications

1. Association à base de cyprotérone et/ou d'un ester de celle-ci hydrosoluble, **caractérisée en ce qu'**elle comprend le mélange de cyprotérone et/ou d'ester de celle-ci avec au moins une cyclodextrine choisie dans le groupe comprenant la bêta-cyclodextrine et ses dérivés, la gamma-cyclodextrine et ses dérivés et leurs mélanges.

2. Association à base de cyprotérone et/ou d'un ester de celle-ci hydrosoluble, **caractérisée en ce qu'**elle comprend le complexe de cyprotérone et/ou d'ester de celle-ci avec au moins une cyclodextrine choisie dans le groupe comprenant la bêta-cyclodextrine et ses dérivés, la gamma-cyclodextrine et ses dérivés et leurs mélanges.

3. Association suivant l'une ou l'autre des revendications 1 et 2, **caractérisée en ce qu'**elle comprend de l'acétate de cyprotérone.

4. Association suivant l'une quelconque des revendications 1 à 3, **caractérisée en ce que** les dérivés de bêta- et gamma- cyclodextrine sont les produits obtenus par éthérification ou estérification d'une ou plusieurs fonctions alcool libres de la cyclodextrine respective.

5. Association suivant la revendication 4, **caractérisée en ce que** le dérivé de bêta-cyclodextrine est choisi dans le groupe comprenant la diméthyl bêta-cyclodextrine, l'hydroxypropyl bêta-cyclodextrine, la bêta-cyclodextrine méthylée de façon aléatoire et la sulfobutyléther 7 bêta-cyclodextrine.

6. Association suivant la revendication 4, **caractérisée en ce que** le dérivé de gamma-cyclodextrine est choisi dans le groupe comprenant la diméthyl gamma-cyclodextrine, l'hydroxypropyl gamma-cyclodextrine et la gamma-cyclodextrine méthylée de façon aléatoire.

7. Association suivant l'une quelconque des revendications 1 à 6, **caractérisée en ce que** le rapport molaire cyprotérone et/ou ester de celle-ci/cyclodextrine est de 1/1 à 1/100.

8. Procédé de préparation d'un complexe à base de cyprotérone et/ou d'ester de celle-ci sous forme liquide suivant l'une quelconque des revendications 2 à 7, **caractérisé en ce qu'**on agite une solution aqueuse de cyclodextrine avec un excès de cyprotérone et/ou d'ester de celle-ci jusqu'à formation d'un complexe en solution et on filtre ladite solution pour récupérer ledit complexe en solution dans le filtrat.

9. Procédé de préparation d'un complexe à base de cyprotérone et/ou d'ester de celle-ci sous forme liquide suivant l'une quelconque des revendications 2 à 7, **caractérisé en ce qu'**on ajoute une solution de cyprotérone et/ou d'ester de celle-ci dans un solvant organique à une solution aqueuse de cyclodextrine et on soumet la solution ainsi obtenue à une agitation suffisante jusqu'à formation d'un complexe soit après évaporation du solvant soit en présence du solvant.

10. Procédé suivant la revendication 9, **caractérisé en ce que** le solvant est choisi dans le groupe comprenant les alcools, l'acétone, les polyols tels que propylène glycol, glycérol et leurs mélanges.

11. Procédé de préparation d'un complexe à base de cyprotérone et/ou d'ester de celle-ci sous forme solide, **caractérisé en ce qu'**on soumet la solution telle qu'obtenue suivant l'une quelconque des revendications 8 à 10, à une lyophilisation ou une nébulisation pour obtenir le complexe sous forme solide.

12. Composition pharmaceutique ayant une activité antiandrogénique et/ou progestative, comprenant une quantité efficace d'une association à base de cyprotérone et/ou d'un ester de celle-ci suivant l'une quelconque des revendications 1 à 7, et plus particulièrement d'une association à base d'acétate de cyprotérone, combinée à au moins un excipient approprié et à d'éventuels autres agents thérapeutiques.

13. Composition pharmaceutique ayant une activité antiséborrhéique et/ou antialopécique, comprenant une quantité efficace d'une association à base de cyprotérone et/ou d'un ester de celle-ci suivant l'une quelconque des revendications 1 à 7, et plus particulièrement d'une association à base d'acétate de cyprotérone, combinée à au moins un excipient approprié et à d'éventuels autres agents thérapeutiques.

14. Composition suivant la revendication 12 ou 13, **caractérisée en ce qu'**elle est sous une forme de dosage destinée à une administration par la voie topique, percutanée, transcutanée ou transmuqueuse.

15. Utilisation de l'association à base de cyprotérone et/ou d'un ester de celle-ci suivant l'une quelconque des revendications 1 à 7 et/ou préparée suivant l'une quelconque des revendications 8 à 11, dans la préparation d'un médicament dans le traitement chez l'homme de l'hypersexualité, de la puberté précoce et du cancer de la prostate et chez la femme, en combinaison ou non avec un oestrogène, dans le traitement de l'hyperandrogénie, de l'hirsutisme, de l'acné sévère et des troubles liés à la ménopause.

16. Utilisation de l'association à base de cyprotérone et/ou d'un ester de celle-ci suivant l'une quelconque des revendications 1 à 7 et/ou préparée suivant l'une quelconque des revendications 8 à 11, dans la préparation d'un médicament dans le traitement de la séborrhée et/ou de l'alopécie androgénique.
